(19) Europäisches Patentamt
European Patent Office
Office européen des brevets

(11) Veröffentlichungsnummer: 0 000 478
B1

(12) **EUROPÄISCHE PATENTSCHRIFT**

(45) Veröffentlichungstag der Patentschrift: 25.11.81

(51) Int. Cl.³: C 07 C 45/00, C 07 C 49/17

(21) Anmeldenummer: 78100262.1

(22) Anmeldetag: 28.06.78

(54) **Verfahren zur Herstellung von Acyloinen.**

(30) Priorität: 20.07.77 DE 2732714

(43) Veröffentlichungstag der Anmeldung: 07.02.79 Patentblatt 79/3

(45) Bekanntmachung des Hinweises auf die Patenterteilung: 25.11.81 Patentblatt 81/47

(84) Benannte Vertragsstaaten: BE CH FR GB NL

(73) Patentinhaber: Henkel Kommanditgesellschaft auf Aktien
-Patentabteilung- Postfach 1100 Henkelstrasse 67
D-4000 Düsseldorf 1 (DE)

(72) Erfinder: Krause, Horst-Jürgen, Dr.
Am Nettchesfeld 22
D-4000 Düsseldorf 13 (DE)

(56) Entgegenhaltungen:

Macromolecules, 1968, 1, Seiten 452 bis 455

J. CHEM. SOC. CHEM. COMM., 891 (1973)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 000 478 B1

Courier Press, Leamington Spa, England.

Verfahren zur Herstellung von Acyloinen

Die Erfindung betrifft ein Verfahren zur Herstellung von Acyloinen durch Kondensieren von Aldehyden in Gegenwart von Thiazoliumsalzen.

Die Herstellung von Acyloinen durch Kondensation von Aldehyden in Gegenwart von niedermolekularen Thiazoliumsalzen als Katalysator und Basen als Co-Katalysator ist literaturbekannt. Entsprechende Reaktionen sind beispielsweise in J. A. Chem. Soc. 80 (1958), 3719—3726, J. Chem. Soc. Chem. Commun. 1973, 891 und Synthesis Commun. 1976, 734 beschrieben.

Die bekannten Verfahren haben den Nachteil, daß die als Katalysatoren eingesetzten Thiazoliumsalze nur mit Hilfe von sehr aufwendigen Methoden aus dem Reaktionsgemisch entfernt werden können. Gelingt es nicht, das rohe Reaktionsprodukt vollständig vom Thiazoliumsalz zu befreien, so entstehen bei der nachfolgenden Destillation unangenehm riechende Zersetzungsprodukte, die zum Teil mit dem Destillat übergehen und die Qualität der erhaltenen Acyloine stark beeinträchtigen. Ein weiterer Nachteil besteht darin, daß die niedermolekularen Thiazoliumsalze nach der Aufarbeitung des Reaktionsgemisches aus dem Waschwasser nur unter großen Schwierigkeiten in reiner Form wiedergewonnen werden können.

Aus Macromolecules, 1968, 1, S. 452—455 ist bekannt, daß Poly-(4-vinylthiazol) und Poly-(4-methyl-5-vinylthiazol) in teilweise quaternierter Form katalytische Aktivität bei der Kondensation von Furfural entfalten. Aus den dort angeführten Ergebnissen muß geschlossen werden, daß eine ausreichende katalytische Wirkung auf die Furoinkondensation nur dann eintritt, wenn der Thiazolkern mit der Polymerkette in 5-Stellung verbunden ist. Die Verwendung von Polyvinylthiazolen als Katalysatoren kommt für technische Verfahren derzeit nicht in Betracht, da die für ihre Herstellung notwendigen monomeren Vinylthiazole nur schwer zugänglich sind.

Der Erfindung lag daher die Aufgabe zugrunde, ein Verfahren zur Herstellung von Acyloinen zu finden, bei dem die beschriebenen Nachteile nicht auftreten. Es sollte insbesondere ein Katalysator gefunden werden, der sich aus leicht zugänglichen Komponenten herstellen und in einfacher Weise aus dem Reaktionsgemisch der Acyloinkondensation entfernen läßt.

Diese Aufgabe wird gelöst durch ein Verfahren zur Herstellung von Acyloinen der Formel

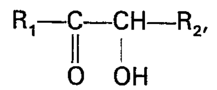 I

in der $R_1$ und $R_2$ gleich oder verschieden sind und geradkettige oder verzweigte Alkylreste mit 1 bis 18 Kohlenstoffatomen, Cycloalkylreste mit 3 bis 18 Kohlenstoffatomen, Arylalkylreste mit 7 bis 18 Kohlenstoffatomen oder $R_1$ und $R_2$ zusammen einen Alkylenrest mit 2 bis 4 Kohlenstoffatomen darstellen, durch Kondensation entsprechender Aldehyde in Gegenwart von Katalysatoren und Basen, das dadurch gekennzeichnet ist, daß man als Katalysatoren Reaktionsprodukte aus Thiazolen der Formel

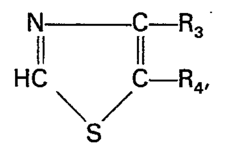 II

in der $R_3$ Wasserstoff oder einen Alkylrest mit 1 bis 5 Kohlenstoffatomen und $R_4$ Wasserstoff, einen Alkylrest mit 1 bis 5 Kohlenstoffatomen oder eine Hydroxyalkalgruppe mit 2 bis 4 Kohlenstoffatomen darstellen, und einem vernetzten Chlormethylpolystyrol verwendet.

Für die Herstellung von Acyloinen nach dem erfindungsgemäßen Verfahren kommen als Ausgangsmaterial Aldehyde der Formel

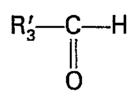

in Betracht. In dieser Formel steht $R_3'$ für einen geradkettigen oder verzweigten Alkylrest mit 1 bis 18 Kohlenstoffatomen, einen Cycloalkylrest mit 3 bis 18 Kohlenstoffatomen, einen Arylalkylrest mit 7 bis 18 Kohlenstoffatomen oder für die Gruppe

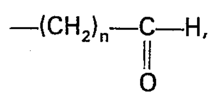

in der n eine ganze Zahl von 2 bis 4 bedeutet.

Als Beispiele für Aldehyde, die in dem erfindungsgemäßen Verfahren als Ausgangsmaterial

Verwendung finden können, sind zu nennen: Acetaldehyd, Acetoxy-acetaldehyd, Propionaldehyd, n-Butyraldehyd, iso-Butyraldehyd, Pivalinaldehyd, Valeraldehyd, iso-Valeraldehyd, Capronaldehyd, Onanthaldehyd, 2-Äthyl-hexanal, Caprylaldehyd, Pelargonaldehyd, Caprinaldehyd, Laurinaldehyd, Myristinaldehyd, Palmitinaldehyd, Stearinaldehyd, Cyclopropan-carbaldehyd, Cyclobutan-carbaldehyd, Cyclopentancarbaldehyd, Cyclohexan-carbaldehyd, Cycloheptan-carbaldehyd, Cyclooctan-carbaldehyd, Cycloheptadecan-carbaldehyd, 2-Methyl-cyclohexan-carbaldehyd-(1), 4-Isopropyl-cyclohexan-carbaldehyd-(1), 1-Äthyl-cyclopentan-carbaldehyd-(1), 1,3-Dimethyl-cyclopentan-carbaldehyd, Phenylacetaldehyd, p-Tolyl-acetaldehyd, 2-Phenyl-propionaldehyd, 3-Phenyl-propionaldehyd, 3-p-Tolylpropionaldehyd, 4-Phenylbutyraldehyd, 2-Phenyl-isobutyraldehyd, 2-Methyl-3-phenyl-propionaldehyd, 2-Methyl-3-p-tolyl-propionaldehyd, 2-Phenyl-valeraldehyd, 4-p-Tolyl-valeraldehyd, 2-Methyl-6-phenyl-hexanal-(1), Cyclohexylphenyl-acetaldehyd, 2-Benzyl-heptanal-(1),(2,4-Diisopropylphenyl)-acetaldehyd, Succindialdehyd, Glutardialdehyd und Adipindialdehyd.

Die Kohlenwasserstoffreste der vorgenannten Aldehyde können auch Substituenten enthalten, beispielsweise Hydroxylgruppen, Ester- oder Äthergruppierungen, solange gewährleistet ist, daß diese Substituenten nicht in störender Weise in die Acyloinkondensation eingreifen.

Zur Herstellung der erfindungsgemäß zu verwendenden Katalysatoren setzt man substituierte Thiazole der Formel II mit vernetzten Chlormethyl-polystyrolen bei erhöhter Temperatur um, vorzugsweise bei 60 bis 100°C. Die Chlormethylpolystyrole sind bekannte, teilweise handelsübliche Substanzen, die durch Chlormethylierung von Polystyrolen mittels Formaldehyd und Chlorwasserstoff hergestellt werden können. Als Beispiele für mögliche Thiazole der Formel II sind zu nennen:

1,3-Thiazol, 4-Methyl-1,3-thiazol, 5-Methyl-1,3-thiazol, 4-Äthyl-1,3-thiazol, 5-Äthyl-1,3-thiazol, 4-tert.-Butyl-1,3-thiazol, 4,5-Dimethyl-1,3-thiazol, 4,5-Diäthyl-1,3-thiazol und 5-(2′-Hydroxyäthyl)-1,3-thiazol. Als besonders geeignet hat sich 5-(2′-Hydroxyäthyl)-4-methyl-1,3-thiazol erwiesen.

Die Thiazole läßt man im allgemeinen in einem indifferenten Lösungsmittel oder in Wasser auf die vernetzten Chlormethyl-polystyrole einwirken. Vorzugsweise kommen dabei aprotische Lösungsmittel, wie z.B. Dimethylformamid, Acetonitril. Dioxan oder Tetrahydrofuran in Frage. Auch überschüssiges Thiazol kann als Lösungsmittel verwendet werden. Es hat sich als zweckmäßig erwiesen, in jedem Fall mit einem stöchiometrischen Überschuß des Thiazols zu arbeiten, um eine praktisch vollständige Umsetzung der Chlormethylgruppen des Polystyrolharzes zu erreichen. Nach der Umsetzung wird der Thiazoliumgruppen enthaltende polymere Katalysator abfiltriert, mit einem indifferenten Lösungsmittel, beispielsweise Äther oder Alkohol, extrahiert und schließlich getrocknet.

Als Co-Katalysatoren kommen anorganische und organische Basen in Betracht, beispielsweise Natriumhydroxid, Kaliumhydroxid, Natriumcarbonat, Natriumbicarbonat, Kaliumcarbonat, Natriumacetat, Kaliumacetat, Dinatriumhydrogenphosphat, Triäthylamin, Triäthanolamin, N-Methylpyrrolidin oder basische Ionenaustauscher.

Die Menge des eingesetzten Katalysators ist nicht kritisch. Im allgemeinen wird das erfindungsgemäße Verfahren mit solchen Mengen des Katalysatorharzes durchgeführt, die — bezogen auf den umzusetzenden Aldehyd — 0,1 bis 20 Mol-%, vorzugsweise 0,5 bis 5 Mol-% quaternäre Thiazoliumgruppen enthalten. Zur Durchführung des Verfahrens genügen aber schon Mengen, die weniger als einem Mol-% entsprechen.

Die Menge der eingesetzten Base ist ebenfalls nicht kritisch. Im allgemeinen setzt man eine den im Katalysator enthaltenen Thiazoliumsalzgruppen äquimolare Menge ein. Vorzugsweise arbeitet man jedoch mit der 2- bis 5-fachen molaren Menge. Beim Arbeiten in wäßrigen oder wäßrig-alkoholischen Lösungen ist die Basenmenge so zu bemessen, daß ein pH-Wert von etwa 7,3 bis etwa 8,5 eingehalten wird.

Bei der Durchführung des erfindungsgemäßen Verfahrens werden die umzusetzenden Aldehyde gegebenenfalls in einem Lösungsmittel gelöst, mit dem darin suspendierten Katalysator bei Raumtemperatur oder bei erhöhter Temperatur solange gerührt, bis der erwünschte Umsetzungsgrad erreicht ist. Mit Rücksicht auf die Empfindlichkeit des Katalysators sollte die Reaktionstemperatur 100°C nicht wesentlich übersteigen.

Wird das erfindungsgemäße Verfahren in Gegenwart eines Lösungsmittels durchgeführt, so kommen polare Lösungsmittel wie Wasser, Methanol, Äthanol, Isopropanol, Dioxan, Tetrahydrofuran, Acetonitril, Dimethylformamid, Dimethylsulfoxid, Hexamethylphosphorsäuretriamid und Pyridin sowie Gemische dieser Lösungsmittel, insbesondere Wasser/Äthanolgemische in Betracht.

Nach Beendigung der Reaktion wird der Katalysator durch Filtration aus dem Reaktionsgemisch abgetrennt. Die flüssigen Anteile des Reaktionsgemisches werden nach Abdestillieren des gegebenenfalls vorhandenen Lösungsmittels nach den in der organischen Chemie üblichen Methoden, wie fraktionierte Destillation und Umkristallisieren, aufgearbeitet.

Der abgetrennte Katalysator kann nach Auswaschen mit einem Lösungsmittel und Trocknen wiederverwendet werden.

In einer speziellen Ausführungsform des erfindungsgemäßen Verfahrens wird das Thiazoliumgruppen enthaltende Katalysatorharz in der Basenform eingesetzt. Bei dieser Arbeitsweise kann ohne zusätzliche Base als Co-Katalysator gearbeitet werden. Die Basen- oder $OH^-$-Form des Katalysators

kann sehr leicht dadurch erhalten werden, daß man die Salz-Form des Thiazoliumgruppen enthaltenden Harzes mit einer wäßrigen Lösung einer Base, wie beispielsweise Natriumhydrogencarbonat solange behandelt, bis in dem Harz keine Chloridionen mehr vorhanden sind.

Die über das erfindungsgemäße Verfahren zugänglichen Produkte sind wertvolle Riech- und Aromastoffe; außerdem stellen sie wertvolle Zwischenprodukte für organische Synthesen dar.

Das erfindungsgemäße Verfahren hat gegenüber den bekannten Arbeitsweisen den Vorteil, daß der verwendete Katalysator in äußerst einfacher Weise, nämlich durch Filtrieren, quantitativ aus dem Reaktionsgemisch entfernt werden kann. Jede Qualitätsverminderung der hergestellten Acyloine durch Zersetzungsprodukte, die beim Abdestillieren des Katalysators entstehen, wird dadurch vermieden.

Die nachfolgenden Beispiele sollen die Erfindung erläutern, sie jedoch nicht darauf beschränken.

Beispiel 1 (Katalysatorherstellung)

40 g vernetztes Chlormethyl-polystyrol mit einem Chlorgehalt von 5,78 Gew.-% wurden in 100 ml Dimethylformamid suspendiert und mit 21,6 g 5-(2'-Hydroxyäthyl)-4-methyl-1,3-thiazol 72 Stunden lang bei 80°C gerührt. Anschließend wurde das Harz abfiltriert, mit Äther extrahiert und im Vakuum bei 40°C/0,01 mbar getrocknet. Das 5-(2'-Hydroxyäthyl)-4-methyl-1,3-thiazoliumgruppen enthaltende Harz hatte einen Chlorgehalt von 3,71 Gew.-%.

Beispiel 2

64,1 g (0,5 Mol) destillierter Caprylaldehyd, 23,8 g (0,025 Mol) Thiazoliumsalz-Katalysator aus Beispiel 1 und 15,2 g (0,15 Mol) Triäthylamin wurden im Stickstoffstrom 7 Stunden bei 90°C gerührt. Anschließend wurde die Mischung mit Äther verdünnt und der Katalysator abfiltriert. Der Katalysator wurde mehrmals mit Äther extrahiert. Aus den vereinigten Äther-Filtraten wurde im Stickstoffstrom unter Normaldruck der Äther und darauf im Wasserstrahlpumpenvakuum nicht umgesetzter Caprylaldehyd und überschüssiges Triäthylamin abdestilliert. Der so erhaltene Rückstand (63,3 g) enthielt nach gaschromatographischer Bestimmung 94,9 Gew.-% Capryloin. Zur weiteren Reinigung wurde das Capryloin aus Benzin (65—95°) umkristallisiert. Es wurden 55 g (86% d.Th.) reines Capryloin vom Schmelzpunkt 38—39°C erhalten.

Beispiel 3

110,0 g (2,5 Mol) frisch destillierter Acetaldehyd, 23,8 g (0,025 Mol) Thiazoliumsalz-Katalysator aus Beispiel 1 und 15,2 g (0,15 Mol) Triäthylamin wurden in einem Autoklaven mit Magnetrührer unter einem vorgegebenen Stickstoffdruck von 6,1 bar 8 Stunden bei 80°C bei 80°C gerührt. Der bei 80°C erhaltene Maximaldruck betrug 7,1 bar. Nach Abkühlen und Öffnen des Autoklaven wurde das Reaktionsgemisch zur leichteren Abtrennung des Katalysators im Äther aufgenommen und der Katalysator abfiltriert. Nach dem Abdestillieren des Äthers wurde der Rückstand unter Kohlendioxid im Wasserstrahlpumpenvakuum über eine Vigreux-Kolonne fraktionierend destilliert. Es wurden 68,1 g (61% d.Th.) reines Acetoin vom Sdp. 37—38°C/16 mbar erhalten.

Beispiel 4

25,6 g (0,2 Mol) frisch destillierter Caprylaldehyd wurde unter Rühren und Kühlen in einem Gemisch aus 50 ml Wasser und 50 ml Äthanol gelöst. In diese Lösung wurde bei Raumtemperatur 9,5 g (0,01 Mol) Thiazoliumsalz-Katalysator aus Beispiel 1 eingerührt. Nach Zugabe von 1 g Dinatriumhydrogenphosphat in 5 ml Wasser wurde der pH-Wert der Lösung mit 1n Natronlauge auf 8,4 eingestellt. Anschließend wurde das Gemisch im Stickstoffstrom und unter intensivem Rühren auf 60°C erwärmt und 7 1/4 Stunden lang bei dieser Temperatur gehalten. Nach dem Abkühlen wurde das Reaktionsgemisch wie in Beispiel 2 aufgearbeitet. Das resultierende rohe Reaktionsprodukt enthielt nach gaschromatographischer Bestimmung 73,2 Gew.-% Capryloin, 2,3 Gew.-% Aldol und 23,5 Gew.-% Caprylaldehyd.

Beispiel 5 (Katalysatorherstellung)

30 g des 5-(2'-Hydroxyäthyl)-4-methyl-1,3-thiazoliumgruppen enthaltenden Harzes aus Beispiel 1 (Chlorid-Form) wurden in 1 l gesättigter Natriumhydrogencarbonat-Lösung eingerührt. Die Suspension wurde in eine Austauschersäule gebracht. Das Harz wurde dann solange mit gesättigter Natriumhydrogencarbonat-Lösung gewaschen, bis das ablaufende Filtrat frei von Chloridionen war. Anschließend wurde der jetzt in der $OH^-$-Form voliegende Katalysator mit Wasser gewaschen und bei 40°C getrocknet.

Beispiel 6

16,0 g (0,13 Mol) destillierter Caprylaldehyd und 6 g (0,007 Mol) des Thiazoliumsalz-Katalysators in der $OH^-$-Form aus Beispiel 5 wurden unter Rühren im Stickstoffstrom auf 60°C erwärmt und 4 Stunden auf dieser Temperatur gehalten. Nach dem Abkühlen wurde das Reaktionsgemisch wie in Beispiel 2 aufgearbeitet. Nach gaschromatographischer Analyse enthielt das rohe Reaktionsprodukt 89,0 Gew.-% Capryloin und 11,0 Gew.-% Caprylaldehyd.

Beispiel 7

25,6 g (0,2 Mol) Caprylaldehyd wurden wie in Beispiel 4 in 100 ml Wasser/Äthanol-Gemisch gelöst. In die Lösung wurden 9,5 g (0,01 Mol) Thiazoliumsalz-Katalysator in der $Cl^-$-Form nach Beispiel 1 eingerührt. Nach Zugabe von 1 g Dinatriumhydrogenphosphat in 5 ml Wasser wurde der pH-Wert der Lösung auf 7,4 eingestellt. Das Gemisch wurde unter Stickstoff auf 60°C erwärmt und 7 1/4 Stunden lang bei dieser Temperatur gehalten. Die Aufarbeitung des Reaktionsgemisches erfolgte wie in Beispiel 2 beschrieben. Nach gaschromatographischer Bestimmung enthielt das rohe Reaktionsprodukt 75 Gew.-% Capryloin, 19,2 Gew.-% Caprylaldehyd, 2,6 Gew.-% Acetal und 3,2 Gew.-% Aldol.

Beispiel 8

36,1 g (0,5 Mol) frisch destillierter n-Butyraldehyd und 9,2 g (0,01 Mol) 5-(2'-Hydroxyäthyl)-4-methyl-1,3-thiazoliumgruppen enthaltendes Harz aus Beispiel 1 in der $OH^-$-Form wurden unter Rühren im Stickstoffstrom 5 Stunden lang auf 70°C erwärmt. Nach dem Abkühlen wurde das Reaktionsgemisch wie in Beispiel 2 aufgearbeitet. Das resultierende rohe Reaktionsprodukt enthielt nach gaschromatographischer Bestimmung 84,5 Gew.-% Butyroin und 15,5 Gew.-% Butyraldehyd.

Das Rohprodukt wurde unter Kohlendioxid im Wasserstrahlpumpenvakuum über eine Vigreux-Kolonne fraktionierend destilliert. Es wurden 24,7 g (69% d.Th.) Butyroin vom Sdp. 86°C/17 mbar erhalten. Dieses Produkt bestand nach gaschromatographischer Analyse zu 100% aus Butyroin.

Beispiel 9

Eine Ionenaustauschersäule aus Glas (Innendurchmesser 1 cm; Länge 60 cm), die mit einem Glasmantel und einer Glasfritte versehen war, wurde mit 20 g des Thiazolium-salz-Katalysators in der $OH^-$-Form nach Beispiel 8 beschickt. Die Säule wurde unter Stickstoff mit Caprylaldehyd gefüllt. Der Säuleninhalt wurde dadurch auf 80°C erwärmt, daß heißes Glycerin durch den Flüssigkeitsmantel geleitet wurde. Am Kopf der Säule wurde unter Stickstoff aus einem Tropftrichter frischer Caprylaldehyd in demselben Maße zugegeben, wie am Fuß der Säule Reaktionsprodukt über einen Glashahn abgezogen wurde. Für das Reaktionsprodukt wurde eine mittlere Verweilzeit von 2 Stunden eingestellt.

Das am Fuße der Austauschersäule abgezogene Reaktionsprodukt bestand nach gaschromatographischer Analyse zu 90 Gew.-% aus Capryloin, der Rest war nicht umgesetzter Caprylaldehyd.

Beispiel 10 (Katalysatorherstellung)

20 g (0,093 Mol) vernetztes Poly-chlormethylstyrol mit einem Chlorgehalt von 16,5 Gew.-% wurden in 40 ml Dimethylformamid suspendiert und mit 30,8 g (0,215 Mol) 5-(2'-Hydroxyäthyl)-4-methyl-1,3-thiazol 24 Stunden lang bei 80°C gerührt. Anschließend wurde das Harz abfiltriert, mit Äther extrahiert und im Vakuum bei 40°C/0,01 mbar getrocknet.

Das 5-(2'-Hydroxyäthyl)-4-methyl-1,3-thiazoliumgruppen enthaltende Harz enthielt 8,61 Gew.-% ionogenes Chlor; der Gesamtchlorgehalt betrug 9,33 Gew.-%.

Beispiel 11 (Katalysatorherstellung)

20 g (0,093 Mol) vernetztes Poly-chlormethylstyrol mit einem Chlorgehalt von 16,5 Gew.-% wurden in 50 ml destilliertem Wasser suspendiert und mit 30,8 g (0,215 Mol) 5-(2'-Hydroxyäthyl)-4-methyl-1,3-thiazol 24 Stunden lang bei 80°C gerührt. Danach wurde das Harz abfiltriert, mit Wasser gewaschen, mit Äthanol extrahiert, um übersüssiges 5-(2'-Hydroxyäthyl)-4-methyl-1,3-thiazol zu entfernen. Das gereinigte Harz wurde im Vakuum bei 40°C/0,01 mbar getrocknet.

Der auf diese Weise erhaltene Harzkatalysator enthielt 7,17 Gew.-% ionogenes Chlor; der Gesamtchlorgehalt betrug 7,68 Gew.-%.

Beispiel 12 (Katalysatorherstellung)

30 g (0,06 Mol) 5-(2'-Hydroxyäthyl)-4-methyl-1,3-thiazoliumgruppen enthaltendes Harz nach Beispiel 11 wurden in 1 l gesättigter Natriumhydrogencarbonatlösung eingerührt. Die Suspension wurde in eine Austauschersäule überführt. Das Harz wurde darin so lange mit gesättigter Natriumhydrogencarbonatlösung gewaschen, bis das ablaufende Filtrat frei von Chloridionen war. Anschließend wurde der jetzt in der $OH^-$-Form vorliegende Katalysator mit Wasser gewaschen und bei 40°C/0,01 mbar getrocknet.

Beispiel 13

110,0 g (2,5 Mol) frisch destillierter Acetaldehyd und 9,7 g (0,021 Mol) des Thiazoliumsalz-Katalysators in der $OH^-$-Form nach Beispiel 12 wurden in einem Autoklaven mit Magnetrührer unter einem vorgegebenen Stickstoffdruck von 3 bar 1 Stunde lang auf 80°C erhitzt. Dabei stieg der Druck auf 8,1 bar an. Nach dem Abkühlen wurde das Reaktionsgemisch zur leichteren Abtrennung das Katalysators mit Äther verdünnt und der Katalysator abfiltriert. Aus dem Filtrat wurde der Äther abdestilliert. Der Rückstand wurde unter Kohlendioxid als Schutzgas im Wasserstrahlpumpenvakuum über eine Vigreux-Kolonne fraktionierend destilliert. Es wurden 93,5 g (85% d.Th.) Acetoin vom Sdp.

39—43°C/17 mbar erhalten. Nach gaschromatographischer Analyse handelte es sich um ein 100%ig reines Produkt.

Beispiel 14

1000 g (22,7 Mol) Acetaldehyd und 214 g (0,449 Mol) des Thiazoliumsalz-Katalysators in der $OH^-$-Form nach Beispiel 12 wurden in einem Autoklaven, der mit einer Einrichtung zur Wasserkühlung und Wasserbeheizung versehen war, unter einem vorgegebenen Stickstoffdruck von 2,3 bar bis zum Anspringen der Reaktion auf 90°C erhitzt. Unter Wasserkühlung wurde sodann die Reaktionstemperatur auf 105°C gehalten, wobei der Druck bis auf 7,6 bar anstieg. Sobald der Druck zurückging, wurden innerhalb von 40 Minuten weitere 2000 g (45,4 Mol) Acetaldehyd aus einem Vorratsautoklaven zudosiert. Dabei wurde die Reaktionstemperatur durch Wasserkühlung auf 90 bis 95°C gehalten. Nach beendeter Zugabe wurden zur Vervollständigung der Reaktion 105 Minuten nachgerührt. Aus dem resultierenden Reaktionsgemisch wurde der Katalysator abfiltriert. Der nicht umgesetzte Acetaldehyd wurde im Wasserstrahlpumpenvakuum abdestilliert. Bei der fraktionierenden Destillation des Rückstandes wurden 1912 g Acetoin vom Sdp. 37 bis 39°C/16 mbar (64% d.Th.) erhalten.

**Patentansprüche**

1. Verfahren zur Herstellung von Acyloinen der Formel

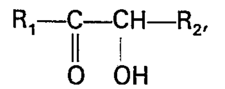

I

in der $R_1$ und $R_2$ gleich oder verschieden sind und geradkettige oder verzweigte Alkylreste mit 1 bis 18 Kohlenstoffatomen, Cycloalkylreste mit 3 bis 18 Kohlenstoffatomen, Arylalkylreste mit 7 bis 18 Kohlenstoffatomen oder $R_1$ und $R_2$ zusammen einen Alkylenrest mit 2 bis 4 Kohlenstoffatomen darstellen, durch Kondensation entsprechender Aldehyde in Gegenwart von Katalysatoren und Basen, dadurch gekennzeichnet, daß man als Katalysatoren Reaktions-produkte aus Thiazolen der Formel

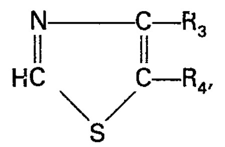

II

in der $R_3$ Wasserstoff oder einen Alkylrest mit 1 bis 5 Kohlenstoffatomen und $R_4$ Wasserstoff, einen Alkylrest mit 1 bis 5 Kohlenstoffatomen oder eine Hydroxyalkylgruppe mit 2 bis 4 Kohlenstoffatomen darstellen, und einem vernetzten Chlormethyl-polystyrol verwendet.

2. Verfahren nach Anspruch 1, dadurch gekennzeichnet, daß man solche Mengen des Katalysatorharzes verwendet, die — bezogen auf den umzusetzenden Aldehyd — 0,1 bis 20 Mol-%, vorzugsweise 0,5 bis 5 Mol-% quaternäre Thiazoliumgruppen enthalten.

3. Verfahren nach den Ansprüchen 1 und 2, dadurch gekennzeichnet, daß man als Katalysator ein Reaktions-produkt aus 5-(2'-Hydroxyäthyl)-4-methyl-1,3-thiazol und einem vernetzten Chlormethylpolystyrol verwendet.

4. Verfahren nach den Ansprüchen 1 bis 3, dadurch gekennzeichnet, daß man das Thiazoliumgruppen enthaltende Katalysatorharz in der Basenform verwendet.

**Claims**

1. A process for the production of acyloins corresponding to the following formula

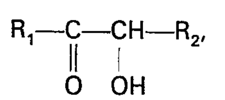

(I)

in which

$R_1$ and $R_2$ are the same or different and represent straight-chain or branched-chain alkyl radicals containing from 1 to 18 carbon atoms, cycloalkyl radicals containing from 3 to 18 carbon atoms, arylalkyl radicals containing from 7 to 18 carbon atoms or $R_1$ and $R_2$ together represent an alkylene radical containing from 2 to 4 carbon atoms, by the condensation of corresponding aldehydes in the presence of catalysts and bases, characterised in that the catalysts used are reaction products of thiazoles corresponding to the following formula

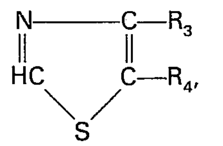

(II)

in which

$R_3$ represents hydrogen or an alkyl radical containing from 1 to 5 carbon atoms and $R_4$ represents hydrogen, an alkyl radical containing from 1 to 5 carbon atoms or a hydroxyalkyl group containing from 2 to 4 carbon atoms, and a crosslinked chloromethyl polystyrene.

2. A process as claimed in Claim 1, characterised in that the catalyst resin is used in quantities containing from 0.1 to 20 mole percent and preferably from 0.5 to 5 mole percent of quaternary thiazolium groups, based on the aldehyde to be reacted.

3. A process as claimed in Claims 1 and 2, characterised in that the catalyst used is a reaction product of 5-(2'-hydroxyethyl)-4-methyl-1,3-thiazole and a crosslinked chloromethyl polystyrene.

4. A process as claimed in Claims 1 to 3, characterised in that the catalyst resin containing thiazolium groups is used in the base form.

**Revendications**

1. Procédé de fabrication d'acyloïnes de formule:

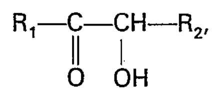

I

dans laquelle $R_1$ et $R_2$ sont identiques ou différents et représentent des radicaux alcoyle à chaîne droite ou ramifiée ayant 1 à 18 atomes de carbone, des radicaux cycloalcoyle ayant 3 à 18 atomes de carbone, des radicaux arylalcoyle ayant 7 à 18 atomes de carbone, ou bien $R_1$ et $R_2$ représentent ensemble un radical alcoylène ayant 2 à 4 atomes de carbone, par condensation des aldéhydes correspondantes en présence de catalyseurs et de bases, caractérisé en ce qu'en tant que catalyseurs on utilise des produits de réaction de thiazols de formule:

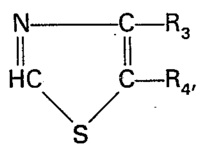

II

dans laquelle $R_3$ représente de l'hydrogène ou un radical alcoyle ayant 1 à 5 atomes de carbone et $R_4$ représente de l'hydrogène, un radical alcoyle ayant 1 à 5 atomes de carbone ou un groupe hydroxyalcoyle ayant 2 à 4 atomes de carbone, et d'un chlorométhylpolystyrène réticulé.

2. Procédé selon la revendication 1, caractérisé en ce qu'on utilise des quantités de résine catalyseur qui — par rapport à l'aldéhyde que l'on fait réagir — contiennent 0,1 à 20 moles %, de préférence 0,5 à 5 moles % de groupes thiazolium quaternaires.

3. Procédé selon les revendications 1 et 2, caractérisé en ce qu'on utilise comme catalyseur un produit de réaction de 5-(2'-hydroxyéthyl)-4-méthyl-1,3-thiazol et d'un chlorométhyl-polystyrène réticulé.

4. Procédé selon les revendications 1 à 3, caractérisé en ce qu'on utilise la résine catalyseur contenant des groupes thiazolium sous la forme de base.